(19) Europäisches Patentamt
European Patent Office
Office européen des brevets

(11) Publication number: **0 211 268 B1**

(12) # EUROPEAN PATENT SPECIFICATION

(45) Date of publication of patent specification: **18.09.91**

(51) Int. Cl.5: **C07D 239/50**, A61K 7/06, A61K 31/505, A61K 9/22, A61K 47/00

(21) Application number: **86109454.8**

(22) Date of filing: **10.07.86**

(54) **Slow-release topical formulations.**

(30) Priority: **19.07.85 IT 2165485**

(43) Date of publication of application: **25.02.87 Bulletin 87/09**

(45) Publication of the grant of the patent: **18.09.91 Bulletin 91/38**

(84) Designated Contracting States: **AT BE CH DE FR GB IT LI LU NL SE**

(56) References cited:
**EP-A- 0 188 793**
**WO-A-83/02558**
**BE-A- 560 809**
**DE-A- 1 944 694**

(73) Proprietor: **Zappia, Vincenzo**
**Via San Giovanni dei Capri, 109/A**
**Napoli(IT)**

Proprietor: **De Rosa, Mario**
**Via Nicolardi, 188**
**I-80131 Napoli(IT)**

(72) Inventor: **Zappia, Vincenzo**
**Via San Giovanni dei Capri, 109/A**
**Napoli(IT)**
Inventor: **De Rosa, Mario**
**Via Nicolardi, 188**
**I-80131 Napoli(IT)**

(74) Representative: **Bianchetti, Giuseppe**
**Studio Consulenza Brevettuale Via Rossini, 8**
**I-20122 Milan(IT)**

**EP 0 211 268 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention concerns new 2,4-diamino-6-piperidino-pyrimidine-3-oxide (minoxidil) salts having hydrosoluble polymeric polyanions, processes for the preparation thereof and therapeutic formulations containing them as the active principle.

The 2,4-diamino-6-piperidino-pyrimidine-3-oxide, commonly known as minoxidil, is a synthetic drug exerting a remarkable vasodilating action. Because of this pharmacological activity the compound is used since 10 years in form of oral formulations in the treatment of resistant hypertensions of any etiology. A drug side-effect is the onset of hypertricosis phenomena in almost all the treated subjects, independently from the age and sex.

In a series of recent publications (Fenton D.A. et al., J. Royal Soc. Med. 75, 963, 1982; Fenton D.A. et al., British Med. J. 287, 1015, 1983; Weiss V.C. et al., Acta Derm. 120, 457, 1984; Wester R.C., J. Invest. Dermatol. 82, 515, 1984; Weiss V.C. Et al., J. Invest. Dermatol. 82, 90, 1984), the effectiveness of the topical use of minoxidil for the therapy of different forms of alopecia has been widely documented.

Although the mechanism of the minoxidil induced hypertricosis is not known in detail, according to one of the most qualified theories, the new growth of the hair seems to be the consequence of an increase of the blood flow at the follicles, connected with the potent vasodilating action of minoxidil.

More recently, it has been shown that the drug acts also as an immunomodulating agent, existing a correlation between the minoxidil treatment and the appearance of perifollicular infiltrates in the cutis area affected by the alopecia.

In the treatments up to now tested, the active principle, dissolved in ternary systems comprising ethanol, isopropanol and water, or dispersed in ointments, is applied on the skin.

The achievement of positive results is connected with:

1) systematic treatments so as to make continuously available the active principle (the present formulations require 3-4 applications per day);

2) long duration of treatment, ranging from 3 to 6 months, according to the kind of the used formulations;

3) preparations of occlusive kind, providing therapeutic effects in shorter times.

On the ground of said experimental findings, an occlusive formulation of minoxidil, able to gradually release, at the skin level, the active principle, represents an ideal solution.

Said formulation would in fact allow the reduction of the applications' frequency, providing in the same way a continuous availability of the active principle in optimal concentrations to exert the action at the cutaneous level on the hair follicles but avoiding contemporaneously undesired hypotensive effects due to the fast absorption and to the high vasodilating activity of the drug, the latter being the most serious obstacle to a wide use of the drug in the treatment of alopecia.

BE-A-560809 describes the preparation of salts of basic antibiotics having polyanions derived from polymeric acids, aiming to reduce their toxicity and improve their therapeutic value through enhanced bioavailability.

It has now been found that the minoxidil salts having hydrosoluble polyanions are endowed with advantageous characteristics, so as to make them particularly suited to the use as slow-release, topical drugs of occlusive kind, with high coating potency, to be used in the treatment or prevention of alopecia or of hairs loss.

The charge state of minoxidil, controlling its water solubility, depends on the medium's pH, because more sites which can be protonated are present in the molecule. At neutral pH, the hydrophobicity of minoxidil is high and its water solubility is extremely low; in acidic media up to pH 4.5, minoxidil is on the contrary very soluble, because it behaves as a polycation, having, according to the medium acidity, up to 4 positive charges, as shown in the following formula:

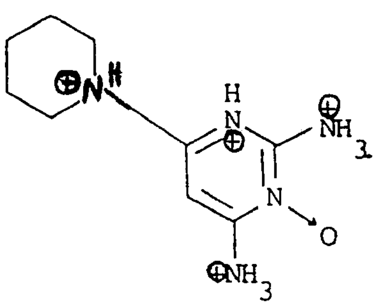

The charge state and the structural peculiarities of minoxidil in aqueous solution at pH up to 5, are such as to provide a stable and specific interaction of the molecule with the negatively charged sites of the hydrosoluble polyanion. Consequence of said interaction is the formation of slightly soluble or soluble salts, characterized by a low value of the dissociation constant.

The stoichiometry of said salts depends from more factors such as: a) the minoxidil-polymeric acid furnishing the polyanion ratio used in the reaction; b) the pH and the kind of the reaction medium; c) the chemical nature of the polyanion. Generally, the preparation of slightly soluble salts is carried out using minoxidil-polymeric acid furnishing the polyanion equivalents ranging from 1 to values even lower than 0.1. The stoichiometry of said salts with respect to a given polyanion is a function of the pH of the precipitation step.

If, for instance, the polyanion derives from a strong acid, pH increases up to 4.5 cause an increase of the minoxidil moles-polyanion equivalents ratio in the salt. The electroneutrality of the precipitated salt is provided by the presence of anions and cations in the reaction medium.

A large number of said salts may be dissolved again provided that further acid furnishing the polyanion is added in the reaction medium. Said behaviour, typical of slightly soluble salts of polyelectrolites with multiple charge ions, clearly differentiates said class of compounds from the conventional slightly soluble salts, wherein the addition of a common ion decreases the solubility.

The minoxidil soluble salts having polymeric polyanions are poorly dissociated and the minoxidil cations firmly interact in water with the negatively charged macromolecule, because of the high cooperation of the electrostatic interactions and the particular situation of the microenvironment on the surface of the uniformely negatively charged polyanion.

In aqueous medium, the soluble and insoluble minoxidil salts with polyanions are characterized by the achievement of equilibrium conditions in which the active principle is mainly present in a form bound to the polyanion and only to a small extent as dissociated ion. In systems wherein the free ion is continuously removed from the equilibrium, as in the case of cutaneous absorption of the drug, a continuous displacement towards the dissociation of the minoxidil-polyanion salt occurs. In other words, the complex behaves like a slow-release system, controlled under the kinetic profile by the characteristics of the absorption process, which in any case may not directly influence the minoxidil-polyanion salt because of the high molecular weight of said compound.

A direct experimental evidence of the slow-release mechanism, characterizing the minoxidil-polyanion salts, is obtained by dialysis equilibrium tests, as shown in the enclosed Figure. Using dialysis membranes impermeable to the polyanion and to the salt thereof with minoxidil, an equilibrium between free and polyanion bound minoxidil is obtained after a while. If the dialysis solution is substituted, the equilibrium is recovered after dissociation of a further amount of salt. This process mimicks "in vitro" the drug's slow release action at the skin level, characterized by a constant and uniform absorption of the active principle, whose availability in time is provided by the continuous dissociation of the salt.

The topical use of the minoxidil hydrosoluble salts with polyanions is extremely advantageous, because the high viscosity and adhesivity of the macromolecules causes a remarkable coating power of the preparation, adhering firmly on the skin, giving thereby rise to long-lasting applications of the occlusive kind.

The structural identity of the polyanions and its molecular weight are the parameters by which the optimization of the coating power of the active principle can be regulated with continuity. Said parameter is extremely important for the pharmacological action, having been already shown that occlusive formulations give therapeutic results in shorter periods of time.

The use of the slightly soluble minoxidil salts with polyanions involves, on the other hand, the use of suitable carrier systems.

Generally, minoxidil salts with polyanions are prepared from strong or weak polyacids. Examples of polyanions deriving from strong polyacids are polymers or copolymers containing in the macromolecular backbone, recurrent $-SO_3^{\ominus}$ groups (polyethylenesulfonate, polystyrenesulfonate), $-OSO_3^{\ominus}$ groups (polyvinylsulfate, polydextransulfate),

$$-O-PO_3^{\ominus}$$

and $-O-PO_3H^{\ominus}$ groups (polyvinylphosphate),

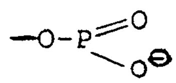

(polyphosphates, metaphosphates). Examples of polyanions deriving from weak polyacids are polymers or copolymers containing $-COO^{\ominus}$ groups (polyacrylates, polymethacrylates, carboxymethylcellulose, polygalacturonates). As far as the polyanion molecular weight is concerned, the formation process of the salt is not critically controlled by said parameter.

It is in fact generally observed that each polymeric acid may form in acidic medium minoxidil salts poorly dissociated in a wide range of molecular weights, range which has an upper limit in the polyanion hydrosolubility and a lower limit in a polymerization degree sufficient to classify the molecule as a polyelectrolyte (Doty P. et al. in "Polymeric Electrolytes", Ann. Rev. Phys. Chem. 3, 81, 1952).

The formation of minoxidil salts with polyanions is carried out by adding to acidic minoxidil solutions (preferred pH from 2.0 to 4.5) a solution of the polymeric acid furnishing the polyanion at the same pH, till the maximum degree of precipitation is obtained. The addition of further amounts of polymeric acid generally causes the dissolution of the precipitate, with the formation of the poorly dissociated soluble salt.

It is possible to carry out the precipitation of minoxidil from extremely diluted ( <1 mM) solutions, but the process is preferably carried out with concentrations ranging from 2 to 200 mM.

The stoichiometry of the slightly soluble minoxidil salts with a certain polyanion may be changed in a certain interval according to the pH of the medium wherein the precipitation is carried out.

The stoichiometry of the poorly dissociated soluble salts depends on the excess of polymeric acid added in order to solubilize again the precipitate.

The formation of the two kinds of salts, insoluble and soluble, may also be carried out adding the minoxidil solution to that of the polymeric acid furnishing the polyanion.

In this case, the poorly dissociated soluble salt, characterized by the higher value of the ratio minoxidil moles/polyanion equivalents, is obtained by quenching the reaction when the formation of the precipitate, at the given pH, begins; further additions of minoxidil cause the quantitative precipitation of the salt.

The pH of the solutions of the minoxidil salts with polyanions may be represented by the following formula:

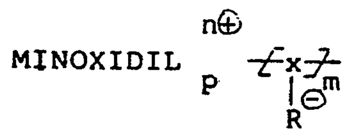

wherein p is the number of molecules of minoxidil associated with each molecule of polyanion, n is ranging from 1 to 4 as a function of the precipitation pH and, by way of an example, the group

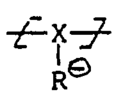 may represent a group of formula 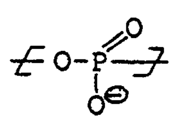 (poly- and metaphosphates), or 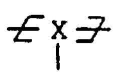 may represent a groups of formula 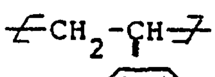 or 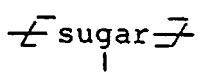 and R one of the following groups 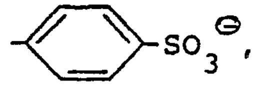, $-SO_3^{\ominus}$, $-OSO_3^{\ominus}$, $-OPO_3H^{\ominus}$, $COO^{\ominus}$.

When the preparation of the salt yields a stoichiometry wherein np≠m, anions or cations in the medium contribute to the maintaining of the salt electroneutrality.

The process described in the present invention, for their convenience and low costs, are particularly suited to the application on industrial phase.

The compounds of the present invention may be formulated in topical, pharmaceutical and cosmetic compositions, using conventional excipients and techniques.

By way of an example, the salts object of the invention may constitute the active principle of ointments,

lotions, creams, shampoos, gels, sprays and balsams, containing suitable carriers and excipients such as detergents, surfactants, parfums, preserving agents and colours. The concentration of the active principle, expressed as minoxidil, ranges from 1 to 10% by weight.

The compositions according to the invention wil be usually administered once a day, allowing a continuous and controlled release of the active principle, with increased effectiveness in comparison with conventional formulations.

The following examples further illustrate the invention.

**EXAMPLE 1**

209 Grams of minoxidil are suspended in 20 liters of distilled water. Under stirring, HCl 1M is added till complete dissolution of minoxidil, taking care that the final pH is 3.5. Water is added up to 25 liters so as to have a resulting 40 mM minoxidil solution. Under strong stirring, this solution is gradually added to 7.5 liters of a 0.4N solution of sodium polyparastyrenesulfonate (Mw 7 x $10^4$), whose pH has been adjusted to 3.5 (the equivalent weight of the polyanion is 183).

The UV spectrum of the solution presents a maximum at 283 nm, characteristic of minoxidil (molar $\epsilon$=14,700), while at lower wavelenghts the second absorption maximum of minoxidil (229 nm, molar $\epsilon$= 29,000) overlaps with the absorption spectrum of the benzene cromophore of the polyanion. The $^1$H-NMR spectroscopy ($^2H_2O$; pH 3.5) of the poorly dissociated salt of minoxidil with polyparastyrenesulfonate shows respectively, in the correct integration ratio, the polyanions signals centered at $\delta$ 7.5 (6H; H 3 and 5 on the benzene ring); 6.5 (6H; H 2 and 6 on the benzene ring); 1.4 (9H; -$CH_2$-CH) and those of minoxidil at $\delta$ 3.0 (2H; H 2' and 6'); 1.2 (1H; H 4'); 1.1 (2H; H 3' and 5'). In the used experimental conditions the proton in 5 on the aromatic ring completely exchanges with the deuterated solvent and the signal is therefore not visible in the $^1$H-NMR spectrum. Said spectroscopical pattern clearly shows that, in solution, the minoxidil salt with polyparastyrenesulfonate is present in non-dissociated form; the signals of the cation, in fact, analogously to what occurs for the polyanion, lose the multiplicity belonging thereto and appear as broad signals, shifted of 0.25 $\delta$ at higher fields with respect to the signals of minoxidil chloride at the same pH. The spectroscopical characterization (UV and $^1$H-NMR) of the UV-absorbing material crossing the dialysis membrane (cut-off limits ~10,000) shows that it is made up of minoxidil ion. The so prepared solution of minoxidil with polyparastyrenesulfonate is remarkably viscous and the identity of the salt remains indefinitely unchanged at room temperature.

**EXAMPLE 2**

The same process described in Example 1 is carried out, using polyparastyrenesulfonate having molecular weight 6 x $10^6$. The spectroscopical characterization of the obtained salt and its behaviour in equilibrium dialysis tests are similar to what reported in the Example 1; the only observed difference is an higher viscosity of the solution of the salt.

**EXAMPLE 3**

The same process as described in Example 1 is carried out, but the pH of the minoxidil and polyparastyrenesulfonate solutions is 4.4. The spectroscopical characterization of the salts obtained and its behaviour in equilibrium dialysis are similar to what reported thereinafter.

**EXAMPLE 4**

69.7 Grams of minoxidil are suspended in 1.5 liters of distilled water. Under stirring, 1M HCl is added till complete dissolution of minoxidil, taking care that the final pH of the solution is 2.4. Water is added up to 2 liters so that the resulting solution has a concentration of 167 mM. Under strong stirring, 0.5 liters of 1.3N sodium polyparastyrenesulfonate (Mw 5 x $10^5$) solution, whose pH has been adjusted to 2.4 (the equivalent weight of the polyanion is 183), are slowly added to said solution. The formation of a milky emulsion is immediately noticed, which has a tendency to coagulate, giving a dense white precipitate which can be recovered by decantation of the solution. After washing with $H_2O$, the precipitate is dried under vacuum, giving 175 g of a white crystalline, non-hygroscopic product, which is finely triturated.

The precipitate, suspended again in 0.5 liters of 0.66N sodium polyparastyrenesulfonate, is easily solubilized, yielding the poorly dissociated salt, whose spectroscopical characterization (UV and $^1$H-NMR) and behaviour in equilibrium dialysis experiences are similar to what reported in Example 1.

Said equilibrium dialysis experience, directly carried out on the suspension of the salt, analogously to what hereinafter exemplified, demonstrate the existence of an equilibrium between minoxidil ion in solution and minoxidil bound to polyanion.

EXAMPLE 5

627 Grams of minoxidil are suspended in 25 liters of distilled water. Under stirring HCl 2N is added up to complete dissolution of minoxidil, taking care that the final pH of the solution is 4.4. Water is added up to 30 liters so that the concentration of minoxidil in the final solution is 0.1N. Under strong stirring, 10 liters of a 0.3N sodium parapolystyrenesulfonate (Mw 5 x $10^5$) solution, pH 4.4 is slowly added to said solution. The formation of a white precipitate which can be removed by decantation is observed.

After washing with water, the precipitate is dried under vacuum giving 1.045 g of a white crystalline solid. The precipitate, suspended again in 20 liters of a solution 0.3N pH 4.4, is easily dissolved, yielding the poorly dissociated salt, whose spectroscopical characterization (UV and $^1$H-NMR) and behaviour in dialysis equilibrium are analogous to what reported for the Example 1. Experiences of equilibrium dialysis, carried out on the salt suspension, show the existence of an equilibrium between minoxidil and minoxidil bound to the polyanion.

EXAMPLE 6

104 Grams of minoxidil are suspended in 9 liters of distilled water. Under stirring HCl 1N is added up to complete dissolution of minoxidil, taking care that the final pH of the solution is 4.4. Water is added up to 10 liters so that the concentration of minoxidil in the final solution is 50 mM. Under strong stirring, 1.5 liters of a 1N sodium polyacrylate (Mw 2.5 x $10^5$) solution, whose pH has been adjusted to 4.4 (the equivalent weight of the polyanion is 71). A white precipitate which can be removed by decantation is obtained.

After washing with water, the precipitate is dried under vacuum giving 190 g of a white crystalline solid which is finely triturated. The spectroscopical characterization (UV and $^1$H-NMR) of the UV-absorbing material crossing the dialysis membrane (cut-off limits ~10,000) shows that it is made up of minoxidil ion, while the solution remaining up stream with respect to the membrane is made up by polyacrylic acid, as shown by $^1$H-NMR spectroscopical data showing, in the correct integration ratios at 1.8 and 2.2 $\delta$, in form of broad signals respectively the methylene and methine groups in the polymeric chain.

EXAMPLE 7

10.4 Grams of minoxidil are suspended in 0.9 liters of distilled water. Under stirring, HCl 1M is added till complete dissolution of minoxidil, taking care that the final pH is 3.2. Water is added up to 1 liter so as to have a resulting 50 mM minoxidil solution. Under strong stirring, this solution is slowly added to 1 liter of a 0.1N solution of hexametaphosphate, pH 3.2 (the equivalent weight of the polyanion is 79). A viscous precipitate, which is recovered by decantation, is obtained.

After washing with $H_2O$ and drying under vacuum, 12 g of a white crystalline solid are obtained. Equilibrium dialysis tests carried out on the salt suspension show the existence of an equilibrium between the minoxidil ion and the polyanion found minoxidil.

EXAMPLE 8

20 Grams of minoxidil are suspended in 9 liters of distilled water. Under stirring, HCl 1N is added till complete dissolution of minoxidil, taking care that the final pH is 4.4. Water is added up to 10 liters so as to have a resulting 0.1N minoxidil solution. Under strong stirring, this solution is gradually added to 5 liters of a 1N solution of sodium polydextransulfate (Mw 5 x $10^5$), whose pH has been adjusted to 4.4 (the equivalent weight of the polyanion is 257). The UV spectrum of the solution presents a maximum at 283 nm, characteristic of minoxidil (molar $\epsilon$ = 14,700), and at 229 nm (molar $\epsilon$= 29,000). The $^1$H-NMR spectroscopy ($^2H_2O$; pH 4.4) of the poorly dissociated salt of minoxidil with polydextransulfate shows respectively, in the correct integration ratio, the polyanions signals in the $\delta$ 3.0-6.0 (30 M) and those of minoxidil at $\delta$ 3.0 (2H; H 2' and 6'); 1.2 (1H; H 4'); 1.1 (2H, H 3' and 5'). Said spectroscopical pattern clearly shows that, in solution, the minoxidil salt with polydextransulfate is present in non-dissociated form. Equilibrium dialysis tests carried out on the salt suspension show the existence of an equilibrium between the minoxidil ion and the polyanion bound minoxidil.

**EXAMPLE 9**

In Figure 1, the moles of minoxidil ion recovered in the dialysis water are removed. 10 ml of a solution containing 0.45 mmoles of minoxidil chloride at pH 4.4 (-Δ-) or of the poorly dissociated minoxidil salt with polyparastyrenesulfonate (Ex. 1, Mw 7 x $10^4$, stoichiometry 1:3 minoxidil moles:polyanion equivalents) at pH 4.4. (-▲-) and 10 ml of a fine suspension containing 0.45 mmoles of the slightly soluble minoxidil salt with polyacrylate (Ex. 6, Mw 2.5 x $10^5$, stoichiometry 1:3 minoxidil moles:polyanion equivalents) at pH 4.4 (-•-) are equilibrated through a dialytic barrier with 130 ml of water at pH 4.4.

When the equilibrium conditions are reached at the points marked by the arrows, the equilibrium solution is removed, substituting it with 130 ml of water, till reestablishment of the new equilibrium conditions. While the minoxidil chloride solution reaches rapidly the equilibrium and about 90% of minoxidil is found outside the dialysis membrane, in the case of the polyanion salts the equilibrium is characterized by the output through the membrane only of a portion of the minoxidil ion, while the remaining portion remains fixed, according to the laws of the chemical equilibrium, in the not permeable, undissociated form.

The complete mobilization of the minoxidil ion from the polyanion salt asks for the continuous removal of the minoxidil ion from the equilibrium, as it occurs in the preceeding experiment, by substituting the equilibrium solution with water and, in the case of the topical administration of the drug, by the cutaneous absorption of the active principle.

The obtained results clearly show the existence of an equilibrium in the dissociation of the minoxidil salt with the polyanion.

**EXAMPLE 10**

| 2% Minoxidil solution | |
|---|---|
| Minoxidil polystryrensulfonate (M.W. 7 x $10^4$) | 7.892 g |
| NaOH 1N | 0.8 ml |
| Nipagine | 0.1 g |
| Nipasol | 0.04 g |
| Distilled $H_2O$ q.s. to | 100 ml |

**EXAMPLE 11**

| 2% Minoxidil solution | |
|---|---|
| Minoxidil polystyrensulfonate (M.W. 6 x $10^5$) | 7.621 g |
| NaOH 1N | 0.8 ml |
| Nipagine | 0.1 g |
| Nipasol | 0.04 g |
| Distilled $H_2O$ q.s. to | 100 ml |

**EXAMPLE 12**

| 6% Minoxidil solution | |
|---|---|
| Minoxidil polystyrensulfonate (M.W. 7 x $10^4$) | 23.676 g |
| NaOH 1N | 2.0 ml |
| Nipagine | 0.1 g |
| Nipasol | 0.04 g |
| Distilled $H_2O$ q.s. to | 100 ml |

EXAMPLE 13

| 6% Minoxidil solution | |
|---|---|
| Minoxidil polystyrensulfonate (M.W. 6 x $10^6$) | 22.863 g |
| NaOH 1N | 2.0 ml |
| Nipagine | 0.1 g |
| Nipasol | 0.04 g |
| Distilled $H_2O$ q.s. to | 100 ml |

## Claims

### Claims for the following Contracting States : BE, CH, DE, FR, GB, IT, LI, LU, NL, SE

**1.** 2,4-Diamino-6-piperidino-pyrimidine-3-oxyde (minoxidil) salts having polymeric polyanions, characterized in that the polymeric polyanions are polyphosphates, metaphosphates, polystyrenesulfonates, polyvinylsulfonates, polyvinylsulfates, polyvinylphosphates, polyacrylates, polymetacrylates or polydextransulfates.

**2.** 2,4-Diamino-6-piperidino-pyrimidine-3-oxyde (minoxidil) salts according to claim 1, characterized in that the stoichiometric ratio is ranging from 0.1 to 1.0 moles minoxidil/equivalent-gram of polymer.

**3.** Slightly soluble salts of 2,4-diamino-6-piperidino-pyrimidine-3-oxyde (minoxidil) according to claims 1 and 2, obtained by precipitation in an acidic medium with polymeric acids furnishing the polymeric polyanions.

**4.** Soluble, poorly-dissociated salts of 2,4-diamino-6-piperidino-pyrimidine-3-oxyde (minoxidil) according to claim 1 or 2 obtained by solubilization by means of addition of an excess of polymeric acid furnishing the polymeric polyanion to the salts prepared according to claim 3 or by means of addition of a minoxidil acidic solution to a solution of the polymeric acid furnishing the polymeric polyanion in the range of the stoichiometric ratios not involving precipitation.

**5.** Process for the preparation of the salts of claims 1-4, by precipitation of minoxidil with hydrosoluble salts of polymeric acids furnishing the polymeric polyanions, characterized in that a minoxidil solution having an acidic pH is mixed, under stirring at room temperature, with a solution of the polymeric acid at the same pH and that the thus obtained precipitate, recovered by decantation, fil-. tration or centrifugation, is washed with water and dried by evaporation under vacuum or lyophilization.

**6.** Process for the preparation of the salts of claim 5, characterized in that a solution of the polymeric acid furnishing the polyanion, at a given pH, is added to a minoxidil acidic solution at the same pH, kept under stirring at room temperature, up to the complete dissolution of the precipitate.

**7.** Process for the preparation of the salts of claim 5, characterized in that a solution of the polymeric acid furnishing the polyanion, at a given pH, is added to a minoxidil acidic solution at the same pH, kept under stirring at room temperature, interrupting the addition before the formation of the precipitate.

**8.** Pharmaceutical and cosmetic compositions containing as active principle at least one of the salts of claims 1-5, in addition to non-toxic, suitable excipients.

**9.** Pharmaceutical and cosmetic compositions according to claim 9, suited for the topical administration in form of lotion, ointment, spray, shampoo, cream, balsam or solution.

### Claims for the following Contracting State : AT

**1.** A process for the preparation of 2,4-diamino-6-piperidino-pyrimidine-3-oxide (minoxidil) salts having polymeric polyanions wherein the polyanions are polyphosphates, metaphosphates, polystyrenesulfonates, polyvinylsulfonates, polyvinylsulfates, polyvinylphosphates, polyacrylates, polymetacrylates or

polydextransulfates, characterized in that a minoxidil solution having an acidic pH is mixed, under stirring at room temperature, with a solution of the polymeric acid furnishing the polymeric polyanion at the same pH and that the thus obtained precipitate, recovered by decantation, filtration or centrifugation, is washed with water and dried by evaporation under vacuum or lyophilization.

2. A process for the preparation of soluble, poorly dissociated salts of 2,4-diamino-6-piperidino-pyrimidine-3-oxide (minoxidil), characterized in that a solution of the polymeric acid furnishing the polymeric polyanion, at a given pH, is added to a minoxidil acidic solution at the same pH, kept under stirring at room temperature, up to the complete dissolution of the precipitate.

3. A process for the preparation of soluble, poorly dissociated salts of 2,4-diamino-6-piperidino-pyrimidine-3-oxide (minoxidil), characterized in that a solution of the polymeric acid furnishing the polymeric polyanion, at a given pH, is added to a minoxidil acidic solution at the same pH, kept under stirring at room temperature, interrupting the addition before the formation of the precipitate.

## Revendications

### Revendications pour les Etats contractants suivants : BE, CH, DE, FR, GB, IT, LI, LU, NL, SE

1. Sels de 2,4-diamino-6-pipéridino-pyrimidine-3-oxyde (minoxidil) avec des polyanions polymeriques, caractérisés en ce que les polyanions polymeriques sont polyphosphates, métaphosphates, polystyrènesulfonates, polyvinylsulfonates, polyvinylsulfates, polyvinylphosphates, polyacrylates, polyméthacrylates ou polydextransulfates.

2. Sels de 2,4-diamino-6-pipéridino-pyrimidine-3-oxyde (minoxidil) selon la revendication 1, caractérisés en ce que le rapport stoechiométrique est situé dans l'intervalle allant de 0,1 à 1,0 moles de minoxidil/équivalent-gramme de polymère.

3. Sels faiblement solubles de 2,4-diamino-6-pipéridino-pyrimidine-3-oxyde (minoxidil) selon les revendications 1 et 2 obtenus par précipitation en milieu acide avec des acides polymériques donnant les polyanions polymeriques.

4. Sels solubles, faiblement dissociés de 2,4-diamino-6-pipéridino-pyrimidine-3-oxyde (minoxidil) selon la revendication 1 on 2, obtenus par solubilisation au moyen d'une addition d'un excès d'acide polymerique donnant le polyanion polimerique aux sels préparés conformément à la revendication 3 ou au moyen d'une addition d'une solution acide de minoxidil à une solution de l'acide polymerique donnant le polyanion polymerique, dans le domaine de rapports stoechiométriques ne provoquant pas une précipitation.

5. Procédé de préparation des sels selon les revendications 1-4, par précipitation du minoxidil avec des sels hydrosolubles d'acides polymeriques donnant les polyanions polymeriques, caractérisé en ce qu'une solution de minoxidil ayant un pH acide est mélangée, sous agitation et à température ambiante, avec une solution de l'acide polymerique de même pH et en ce que le précipité ainsi obtenu, récupéré par décantation, filtration ou centrifugation, est lavé à l'eau et séché par évaporation sous vide ou lyophilisation.

6. Procédé de préparation des sels selon la revendication 5, caractérisé en ce qu'une solution de l'acide polymerique donnant le polyanion, de pH donné, est additionnée à une solution acide de minoxidil de même pH, maintenue sous agitation à température ambiante, jusqu'à dissolution complète du précipité.

7. Procédé de préparation des sels selon la revendication 5, caractérisé en ce qu'une solution de l'acide polymerique donnant le polyanion, de pH donné, est additionnée à une solution acide de minoxidil de même pH, maintenue sous agitation à température ambiante, l'addition étant interrompue avant la formation du précipité.

8. Compositions pharmaceutiques et cosmétiques contenant, à titre de principe actif, au moins l'un des sels selon les revendications 1-5, et des excipients appropriés non-toxiques.

9. Compositions pharmaceutiques et cosmétiques selon la revendication 8, appropriées à l'administration

topique sous la forme de lotion, onguent, spray, shampooing, crème, baume ou solution.

**Revendications pour l'Etat contractant suivant : AT**

1. Procédé de preparation de sels de 2,4-diamino-6-pipéridino-pyrimidine-3-oxyde (minoxidil) avec des polyanions pclymeriques, les polyanions polymeriques étant polyphosphates, métaphosphates, polystyrènesulfonates, polyvinylsulfonates, polyvinylsulfates, polyvinylphosphates, polyacrylates, polyméthacrylates ou polydextransulfates, caractérisé en ce qu'une solution de minoxidil ayant un pH acide est mélangée, sous agitation et à température ambiante, avec une solution de l'acide polymerique donnant le polyanion polymerique de même pH et en ce que le précipité ainsi obtenu, récupéré par décantation, filtration ou centrifugation, est lavé à l'eau et séché par évaporation sous vide ou lyophilisation.

2. Procédé de preparation de sels solubles, faiblement dissociés de 2,4-diamino-6-pipéridino-pyrimidine-3-oxyde (minoxidil), caractérisé en ce qu'une solution de l'acide polymerique donnant le polyanion, de pH donné, est additionnée à une solution acide de minoxidil de même pH, maintenue sous agitation à température ambiante, jusqu'à dissolution complète du précipité.

3. Procédé de préparation des sels solubles, faiblement dissociés de 2,4-diamino-6-pipéridino-pyrimidine-3-oxyde (minoxidil) caractérisé en ce qu'une solution de l'acide polymerique donnant le polyanion polymerique, de pH donné, est additionnée à une solution acide de minoxidil de même pH, maintenue sous agitation à température ambiante, l'addition étant interrompue avant la formation du précipité.

**Patentansprüche**
**Patentansprüche für folgende Vertragsstaaten : BE, CH, DE, FR, GB, IT, LI, LU, NL, SE**

1. 2,4-Diamino-6-piperidino-pyrimidin-3-oxid (Minoxidil)-Salze mit polymeren Polyanionen, dadurch gekennzeichnet, daß die polymere Polyanionen Polyphosphate, Metaphosphate, Polystyrolsulfonate, Polyvinylsulfonate, Polyvinylsulfate, Polyvinylphosphate, Polyacrylate, Polymetacrylate oder Polydextransulfate sind.

2. 2,4-Diamino-6-piperidino-pyrimidin-3-oxid (Minoxidil)-Salze nach Anspruch 1 dadurch gekennzeichnet, daß das stöchiometrische Verhältnis von 0,1 bis 1,0 Mol Minoxidil/Grammäquivalent des Polymeren reicht.

3. Leicht lösliche Salze von 2,4-Diamino-6-piperidino-pyrimidin-3-oxid (Minoxidil) nach Anspruch 1 oder 2, erhalten durch Ausfällung in saurer Umgebung mit polymeren Säuren die polymere Polyanionen liefern.

4. Lösliche, wening dissoziierte Salze von 2,4-Diamino-6-piperidino-pyrimidin-3-oxid (Minoxidil) nach Anspruch 1 oder 2 erhalten durch Solubilisation mittels Zugabe eines Überschusses von polymeren, polymere Polyanionen liefernden Säure zu den Salzen, hergestellt nach Anspruch 4, oder mittels Zugabe einer Minoxidil sauren Lösung zu einer Lösung von der polymeren, polymere Polyanionen liefernden Säure, in dem Bereich der stöchiometrischen Verhältnisse, die nicht Ausfällung einbeziehen.

5. Verfahren zur Herstellung der Salze nach einem oder mehreren der Ansprüche 1 bis 4, durch Ausfällung von Minoxidil mit wasserlöslichen Salzen von polymeren, polymere Polyanionen liefernden Säuren, dadurch gekennzeichnet, daß eine Minoxidillösung mit einem sauren pH unter Rühren und bei Raumtemperatur mit einer Lösung der polymeren Säure bei dem gleichen pH-Wert gemischt wird und daß das so erhaltene Präzipitat, erhalten durch Dekantieren, Filtration oder Zentrifugation, mit Wasser gewaschen wird und durch Eindampfen unter Vakuum oder Lyophilisation getrocknet wird.

6. Verfahren zur Herstellung der Salze von Anspruch 5, dadurch gekennzeichnet, daß eine Lösung der polymeren, das Polyanion liefernden Säure bei gegebenem pH zu einer Minoxidil sauren Lösung bei dem gleichen pH gegeben wird, unter Rühren bei Raumtemperatur gehalten wird, bis zur völligen Auflösung des Niederschlags.

7. Verfahren zur Herstellung der Salze nach Anspruch 5, dadurch gekennzeichnet, daß eine Lösung der polymeren, das Polyanion liefernden Säure bei gegebenem pH zu einer Minoxidil sauren Lösung bei dem gleichen pH gegeben wird, unter Rühren bei Raumtemperatur gehalten wird, wobei die Zugabe

vor der Bildung des Niederschlags unterbrochen wird.

8. Pharmazeutische und kosmetische Zusammensetzungen, die als aktives Prinzip wenigstens eins der Salze der Ansprüche 1 bis 5 enthalten, zusätzlich zu nicht toxischen, geeigneten Trägermaterialien.

9. Pharmazeutische und kosmetische Zusammensetzungen nach Anspruch 8, geeignet zur topischen Verabreichung in Form einer Lotion, Salbe, Spray, Shampoo, Creme, Balsam, oder Lösung.

**Patentansprüche für folgenden Vertragsstaat : AT**

1. Verfahren zur Herstellung von 2,4-Diamino-6-piperidino-pyrimidin-3-oxid (Minoxidil)-Salzen mit polymeren Polyanionen, wobei die Polyanionen, Polyphosphat, Metaphosphate, Polystyrolsulfonate, Polyvinylsulfonate, Polyvinylsulfate, Polyvinylphosphate, Polyacrylate, Polymetracrylate oder Polydextransulfate sind, dadurch gekennzeichnet, daß eine Minoxidillösung mit einem sauren pH unter Rühren und bei Raumtemperatur mit einer Lösung der polymeren, polymere Polyanionen liefernden Säure bei gleichem pH gemischt wird und daß der so erhaltene Niederschlag, erhalten durch Dekantierung, Filtration oder Zentrifugation, mit Wasser gewaschen wird und durch Eindampfen unter Vakuum oder Lyophilisation getrocknet wird.

2. Verfahren zur Herstellung von löslichen, schwach dissoziierten Salzen von 2,4-Diamino-6-piperidino-pyrimidin-3-oxid (Minoxidil), dadurch gekennzeichnet, daß eine Lösung der polymeren, polymere Polyanionen liefernden Säure bei gegebenem pH zu einer Minoxidil sauren Lösung bei dem gleichen pH gegeben wird, unter Rühren bei Raumtemperatur gehalten wird, bis zur vollständigen Auflösung des Niederschlags.

3. Verfahren zur Herstellung von löslichen, schwach dissoziierten Salzen von 2,4-Diamino-6-piperidino-pyrimidin-3-oxid (Minoxidil), dadurch gekennzeichnet, daß eine Lösung der polymeren, polymere Polyanionen liefernden Säure bei gegebenem pH zu einer Minoxidil sauren Lösung bei dem gleichen pH gegeben wird, unter Rühren bei Raumtemperatur gehalten wird, wobei die Zugabe vor der Bildung des Niederschlags unterbrochen wird.

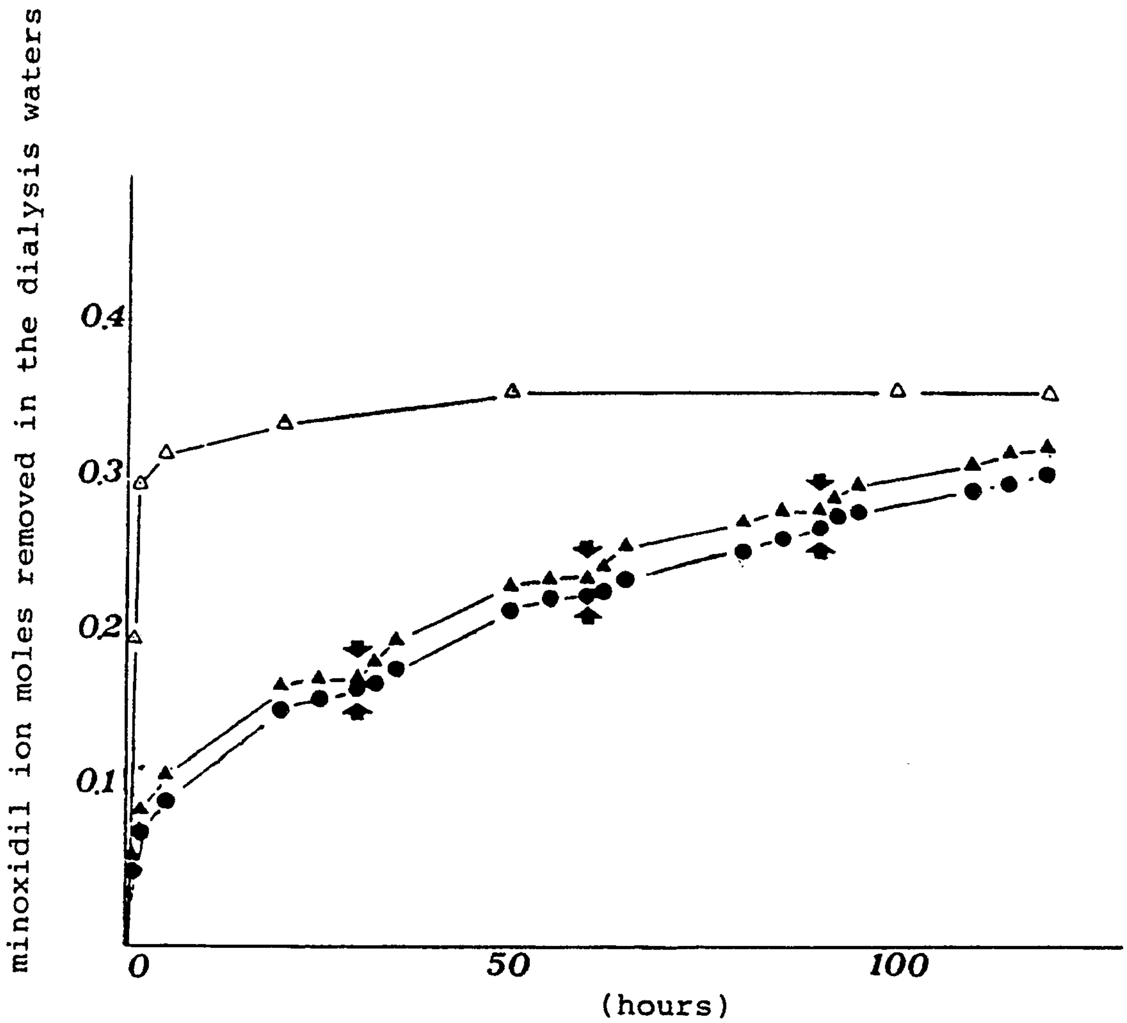
minoxidil ion moles removed in the dialysis waters
0.4
0.3
0.2
0.1
0
50
100
(hours)